Europäisches Patentamt

European Patent Office

Office européen des brevets

(11) Veröffentlichungsnummer: **0 025 890**
**B1**

(12) EUROPÄISCHE PATENTSCHRIFT

(45) Veröffentlichungstag der Patentschrift:
**13.04.83**

(21) Anmeldenummer: **80105116.0**

(22) Anmeldetag: **28.08.80**

(51) Int. Cl.³: **C 07 C  29/03**, C 07 C  31/18,
C 07 C  31/42

(54) Verfahren zur Hydroxylierung von kürzerkettigen aliphatischen Mono- oder Diolefinen.

(30) Priorität: **19.09.79  DE 2937840**

(43) Veröffentlichungstag der Anmeldung:
**01.04.81 Patentblatt 81/13**

(45) Bekanntmachung des Hinweises auf die Patenterteilung:
**13.04.83 Patentblatt 83/15**

(84) Benannte Vertragsstaaten:
**AT BE CH FR GB IT LI NL SE**

(56) Entgegenhaltungen:
**FR-A-1 047 126**
**GB-A-634 118**
**US-A-2 492 201**

(73) Patentinhaber: **Degussa Aktiengesellschaft,
Weissfrauenstrasse 9, D-6000 Frankfurt am Main 1 (DE)**

(72) Erfinder: **Käbisch, Gerhard, Dr., Palmstrasse 1,
D-7888 Rheinfelden (DE)**
Erfinder: **Malitius, Horst, Blümleacker 5,
D-7888 Rheinfelden (DE)**
Erfinder: **Raupach, Siegfried, Langentalstrasse 24,
D-7888 Rheinfelden (DE)**
Erfinder: **Trübe, Rudolf, Ernst-Reuter-Strasse 22,
D-7888 Rheinfelden (DE)**
Erfinder: **Wittmann, Hans, Katzenbuckelweg 12,
D-7888 Rheinfelden (DE)**

## Verfahren zur Hydroxylierung von kürzerkettigen aliphatischen Mono- oder Diolefinen

Die Erfindung betrifft ein Verfahren zur Hydroxylierung von 3-Chlor-2-methylpropen-1, von geradkettigen oder verzweigten, unsubstituierten oder bereits durch 1 bis 2 Hydroxylgruppen substituierten Monoolefinen mit 4 bis 7 Kohlenstoffatomen und endständiger oder innenständiger Doppelbindung, von geradkettigen oder verzweigten, unsubstituierten oder bereits durch 1 bis 2 Hydroxylgruppen substituierten Monoolefinen mit 8 bis 10 Kohlenstoffatomen und innenständiger Doppelbindung oder von geradkettigen oder verzweigten Diolefinen mit 4 bis 10 Kohlenstoffatomen.

Unter Hydroxylierung ist zu verstehen, daß an die olefinischen Doppelbindungen jeweils zwei Hydroxylgruppen angelagert werden. Bei der Hydroxylierung der Monoolefine entstehen demnach vicinale Diole, sofern bereits eine oder zwei Hydroxylgruppen vorhanden sind, die entsprechenden Tri- bzw. Tetrole, bei der Hydroxylierung der Diolefine entstehen ungesättigte Diole bzw. gesättigte Tetrole.

Aus der US-A-2 492 201 ist es bereits bekannt, höhere, gegebenenfalls substituierte Olefine durch Umsetzung mit Ameisensäure und Wasserstoffperoxid zu hydroxylieren. Die Umsetzung erfordert den Einsatz einer relativ großen Ameisensäuremenge, was dazu führt, daß zunächst die entsprechenden Diolmonoformiate gebildet werden, die anschließend in einer zusätzlichen Verfahrensstufe hydrolysiert werden müssen.

Aus der FR-A-1 047 126 ist es ferner bereits bekannt, ungesättigte höhere Fettalkohole durch Umsetzung mit Ameisensäure und Wasserstoffperoxid zu hydoxylieren. Auch dieses bekannte Verfahren erfordert den Einsatz einer Relativ großen Ameisensäuremenge und führt infolgedessen zunächst wieder zu den entsprechenden Diolmonoformiaten.

Schließlich ist in der GB-A-634 118 die Hydroxylierung von Allylalkohol und Octen-1 mittels Ameisensäure und Wasserstoffperoxid beschrieben. Im Falle des Allylalkohols sind bei Einsatz relativ geringer Ameisensäuremengen untragbar lange Reaktionszeiten erforderlich, die nur durch eine starke Erhöhung der Ameisensäuremengen verkürzt werden können. Im Falle des Octen-1 erfordert eine kurze Reaktionszeit den Einsatz eines Wasserstoffperoxids von extrem hoher Konzentration.

Das erfindungsgemäße Verfahren ist dadurch gekennzeichnet, daß man das zu hydroxylierende Olefin bei einer Temperatur zwischen 30 und 80° C mit weniger als 2 Mol Ameisensäure und weniger als 2 Mol Wasserstoffperoxid, jeweils pro Mol zu hydroxylierender Doppelbindung, umsetzt, Ameisensäure mit einer Konzentration zwischen 20 und 100 Gewichtsprozent und Wasserstoffperoxid mit einer Konzentration von weniger als 50 Gewichtsprozent einsetzt, und die Konzentration des Wasserstoffperoxids in der wäßrigen Phase des Reaktionsgemisches während der Gesamtdauer der Umsetzung unter 15 Gewichtsprozent hält.

Vorzugsweise wird die Umsetzung bei einer Temperatur zwischen 45 und 60°C vorgenommen. Die Ameisensäure wird vorteilhafterweise in einer Menge von 0,2 bis 0,8 Mol pro Mol zu hydroxylierender Doppelbindung, das Wasserstoffperoxid vorteilhafterweise in einer Menge von 1,1 bis 1,5 Mol pro Mol zu hydroxylierender Doppelbindung eingesetzt. Bevorzugt wird ein Wasserstoffperoxid mit einer Konzentration von 15 bis 40 Gewichtsprozent verwendet.

Als Ausgangsmaterialien für das erfindungsgemäße Verfahren dienen 3-Chlor-2-methylpropen-1, geradkettige Monoolefine mit 4 bis 7 Kohlenstoffatomen, wie Buten-1, Buten-2, Penten-1, Hexen-1, Hepten-1 oder Hepten-3 und verzweigtkettige Monoolefine mit 4 bis 7 Kohlenstoffatomen, wie 2-Methylpropen-1, 3-Methylbuten-1, 3,3-Dimethylbuten-1, 2,3-Dimethylbuten-1, 2,3-Dimethylbuten-2, 2-Methylpenten-1, 2-Methylpenten-2, 3-Methylpenten-2 oder 2-Äthylbuten-1.

Die einzusetzenden Monoolefine können auch bereits durch 1 bis 2 Hydroxylgruppen substituiert sein. Beispiele für solche Substanzen sind Buten-1-ol-3, Buten-2-ol-1 (Crotylalkohol), Buten-2-diol-1,4, 3-Methylbuten-3-ol-1, 3-Methylbuten-2-ol-1 oder 2-Methylbuten-3-ol-2. Während bei den Monoolefinen mit maximal 7 Kohlenstoffatomen die Doppelbindung an beliebiger Stelle, also am Kettenende oder im Inneren der Kette, stehen kann, sind Monoolefine mit 8 bis 10 Kohlenstoffatomen der Umsetzung nach dem erfindungsgemäßen Verfahren nur dann zugänglich, wenn die Doppelbindung im Inneren der Kette steht. Beispiele für solche Substanzen sind 2,4,4-Trimethylpenten-2, 1,2-Di-(tert.butyl)-äthylen oder Decen-5. Auch die Monoolefine mit 8 bis 10 Kohlenstoffatomen können bereits durch 1 bis 2 Hydroxylgruppen substituiert sein, wie im 2-Äthylhexen-2-ol-1. Schließlich können nach dem erfindungsgemäßen Verfahren auch geradkettige oder verzweigte Diolefine mit 4 bis 10 Kohlenstoffatomen, wie Butadien, Isopren, Hexadien-1,5 oder Decadien-1,9, zu den entsprechenden Tetrolen umgesetzt werden.

Das erfindungsgemäße Verfahren wird vorzugsweise bei Normaldruck durchgeführt. Da jedoch zur Erzielung einer tragbaren Reaktionsgeschwindigkeit die Reaktionstemperatur nicht unter 30° C liegen sollte, versteht es sich von selbst, daß bei Olefinen, deren Siedepunkt unter 30° C liegt, die Anwendung von Überdruck erforderlich ist. In diesem Falle ist es dann zweckmäßig, die Reaktionstemperatur so zu wählen, daß der Druck 10 bar nicht überschreitet. Die Umsetzungen nach dem erfindungsgemäßen Verfahren verlaufen überraschenderweise trotz der verhältnismäßig milden Reaktionsbedingungen sehr glatt und führen innerhalb tragbarer Reaktionszeiten zu hohen Ausbeuten an den gewünschten Di-, Tri- oder Tetrolen.

Bei der praktischen Durchführung des erfindungsgemäßen Verfahrens wird vorzugsweise die gesamte einzusetzende Ameisensäure zusammen mit dem Olefin vorgelegt und das Wasserstoffperoxid langsam zudosiert. Es ist jedoch auch möglich, zunächst nur einen Teil, beispielsweise etwa ein Drittel der Gesamtmenge des zu hydroxylierenden Olefins, zusammen mit der Gesamtmenge an Ameisensäure vorzulegen und das Wasserstoffperoxid und die Restmenge des zu hydroxylierenden Olefins langsam zuzudosieren. In jedem Falle wird das Reaktionsgemisch intensiv gerührt. Zur Verbesserung des Umsatzes empfiehlt sich eine angemessene Nachreaktionszeit, nachdem alle Reaktionsteilnehmer im Reaktionsgefäß vereinigt sind.

Nach beendeter Umsetzung liegen die Reaktionsprodukte üblicherweise in der wäßrigen Phase gelöst vor. Für manche Zwecke können derartige wäßrige Lösungen direkt weiterverwendet werden. In den meisten Fällen wird sich aber nicht nur der Restgehalt an Wasserstoffperoxid störend auswirken, sondern es ist auch erwünscht, daß die gebildeten Di-, Tri- bzw. Tetrole in reinerer Form isoliert werden. Dann kommen für die Aufarbeitung verschiedene Maßnahmen in Betracht.

Falls sich aus dem Reaktionsgemisch eine zweite Phase abscheidet, kann zunächst eine Phasentrennung vorgenommen werden. In vielen Fällen ist es dann ratsam, das praktisch nur in der wäßrigen Phase enthaltene Wasserstoffperoxid weitgehend zu zersetzen. Die Zersetzung kann durch längeres Stehenlassen bei höherer Temperatur (Digestion) oder durch die Einwirkung eines geeigneten Katalysators (z. B. Überleiten über einen Platin-Festbett-Kontakt) oder durch eine Kombination dieser beiden Maßnahmen bewirkt werden.

Nun wird zweckmäßigerweise das Reaktionsgemisch neutralisiert und mit einem geeigneten Lösungsmittel, beispielsweise Äthylacetat, extrahiert. Aus dem Extrakt wird das Lösungsmittel abdestilliert und der verbleibende Rückstand an rohem Di-, Tri- bzw. Tetrol gegebenenfalls durch Erwärmen unter vermindertem Druck von noch enthaltenem Wasser befreit. Alternativ kann auch auf die Neutralisation der im Reaktionsgemisch enthaltenen Ameisensäure verzichtet werden. Dann ist es zweckmäßig, entweder die Eindickung nach Art einer Wasserdampfdestillation kontinuierlich vorzunehmen und so lange fortzuführen, bis das Destillat nur noch geringe Mengen an Ameisensäure enthält. Oder die Ameisensäureabtreibung wird diskontinuierlich derart vorgenommen, daß man den Rückstand nach dem Abtreiben der Hauptmenge an Ameisensäure nochmals mit Wasser versetzt, erneut eindickt und diese Operation mehrmals wiederholt. Der verbleibende Rückstand an rohem Di-, Tri- bzw. Tetrol kann dann wiederum durch Erwärmen unter vermindertem Druck von noch enthaltenen flüchtigen Anteilen befreit werden.

Die so erhaltenen rohen Di-, Tri- bzw. Tetrole liegen mit Reinheitsgraden von etwa 88 bis 96% vor. Sofern eine noch weitergehende Reinigung erforderlich ist, kann diese in üblicher Weise durch Umkristallisation oder durch fraktionierte Destillation unter vermindertem Druck bewirkt werden.

Das erfindungsgemäße Verfahren wird durch die nachfolgenden Beispiele näher erläutert. Prozentangaben bedeuten, sofern nichts anderes angegeben ist, in allen Fällen Gewichtsprozente.

### Beispiel 1

1 Mol Crotylalkohol (72 g) wurde in einer Rührapparatur mit aufgesetztem Rückflußkühler mit 0,5 Mol Ameisensäure (98%ig; 23 g) sowie mit 1,5 Mol $H_2O_2$ (15%ig; 340 g) vermischt und bei 60° C 8 Stunden lang gerührt. Das System war 1-phasig und enthielt 3,9% $H_2O_2$. Der Ansatz wurde über eine 30 cm lange NW 40-Kolonne geleitet, die mit 100 ml Festbettkatalysator (0,1% Platin auf Berlsätteln) gefüllt war. Nach dem Durchleiten konnte $H_2O_2$ analytisch nicht mehr nachgewiesen werden.

Der Ansatz wurde danach 2 Stunden am Rückfluß gekocht und im Vakuum (bis 100° C Sumpftemperatur bei 12 Torr) eingedickt. Es verblieb ein Rückstand von 102 g, der anschließend im Hochvakuum (0,1 Torr) destilliert wurde. Die zwischen 125—130° C übergehende Fraktion (=92 g) bestand aus Methylglycerin.

### Beispiel 2

In einer Apparatur gemäß Beispiel 1 wurde 1 Mol 3-Chlor-2-Methyl-Propen-1 (91 g) mit 0,75 Mol Ameisensäure (98%ig; 35 g) vermischt und bei 60° C mit 1,5 Mol $H_2O_2$ (40%ig; 128 g) im Verlauf von 6 Stunden versetzt. Nachreaktion: 4 Stunden bei 60° C. Der Ansatz wurde — wie in Beispiel 1 beschrieben — katalytisch von $H_2O_2$ befreit und destillativ eingedickt. Bei einer Sumpftemperatur von 100° C (12 Torr) verblieb ein Rückstand (125 g), der anschließend bei 12 Torr totaldestilliert wurde. Die zwischen 103—107° C übergehende Fraktion (103 g) bestand aus 3-Chlor-2-Methyl-Propandiol-1,2.

### Beispiel 3

In einer Apparatur gemäß Beispiel 1 wurden 1 Mol Penten-1 (70 g) mit 1 Mol Ameisensäure vermischt und bei Siedekühlung (ca. 30° C) im Verlauf von 8 Stunden mit 1,5 Mol $H_2O_2$ (40%ig; 128 g) versetzt. Nach einer Nachreaktionszeit von 8 Stunden war das System 1-phasig geworden. Es wurde katalytisch $H_2O_2$-frei gemacht, mit 90 g NaOH (40%ig) neutralisiert und viermal mit je 100 ml Äthylacetat extrahiert.

Die Äthylacetat-Extrakte wurden vereinigt und destillativ vom Lösungsmittel befreit. Es hinterblieb ein Pentan-Diol-Rückstand (roh) von 84 g, dessen Menge sich dadurch weiter erhöhen ließ, daß die wäßrige Phase auf etwa die Hälfte eingeengt und erneut erschöpfend mit Äthylacetat extrahiert wurde.

Bei der Totaldestillation (12 Torr) ergaben 84 g Rohdiol eine Menge von 78 g reinem Pentandiol-1,2, das bei 115—117°C überging.

Der Versuch wurde in einer veränderten Rührapparatur wiederholt, die nach dem Rückflußkühler mit einem Druckhalteventil ausgestattet war, das sich bei einem Überdruck von 2 atü öffnete. Dadurch konnte die Penten-1-Hydroxylierung mit den oben angegebenen Einsatzmengen bei einer Temperatur von 50°C durchgeführt werden. Unter diesen Bedingungen konnte sowohl die $H_2O_2$-Eindosierungszeit als auch die Nachreaktionszeit auf jeweils die Hälfte verkürzt werden. Die Aufarbeitung des bei schwach erhöhtem Druck durchgeführten Versuches ergab praktisch die gleichen Ausbeuten wie bei dem drucklosen Versuch.

### Beispiel 4

In einer Apparatur gemäß Beispiel 1 wurden 1 Mol 2,3-Dimethyl-Buten-2 (84 g) mit 0,6 Mol Ameisensäure (98%ig; 28 g) vermischt und bei 55°C im Verlauf von 4 Stunden mit 1,5 Mol $H_2O_2$ (20%ig; 255 g) versetzt. Nach einer Nachreaktionszeit von 4 Stunden war das System zwar 1-phasig geworden, es konnten jedoch noch 4,2 g Olefin destillativ entfernt werden (Umsatz = 95%).

Nach der üblichen katalytischen $H_2O_2$-Zersetzung wurde der Ansatz mit 50 g NaOH (25%ig) neutral gestellt und viermal hintereinander mit je 100 ml Äthylacetat extrahiert. Die verbleibende wäßrige Phase wurde destillativ auf die Hälfte eingeengt und erneut viermal mit je 50 ml Äthylacetat extrahiert. Alle Äthylacetat-Extrakte wurden vereinigt und zunächst durch Aceotrop-Destillation von noch gelöstem Wasser befreit. Nach Abdestillation der Leichtsieder hinterblieb ein Sumpfprodukt (98 g; Smp. 38—41°C), das einen Diol-Gehalt (Pinakol) von 98% aufwies (Ausbeute 87%).

### Beispiel 5

In einer Apparatur gemäß Beispiel 1 wurden 1 Mol Hepten-3 (98 g) mit 1 Mol Ameisensäure (98%ig; 47 g) bei 55°C im Verlauf von 5 Stunden mit 1,5 Mol $H_2O_2$ (35%ig; 146 g) versetzt. Auch nach einer Rührzeit von 2 Stunden bei 60°C blieb der Ansatz 2-phasig. Der Ansatz wurde katalytisch von Rest-$H_2O_2$ befreit und mit 85 g NaOH (40%ig) neutral gestellt. Die wäßrige Phase wurde viermal mit Äthylacetat (je 100 ml) extrahiert, auf die Hälfte eingeengt und erneut zweimal mit Äthylacetat extrahiert. Alle Äthylacetat-Extrakte wurden mit der organischen Phase vereinigt und destillativ von den Leichtsiedern befreit. Es verblieb ein Sumpfprodukt (Roh-Heptandiol-3,4 = 132 g), das durch Feinfraktionierung ($Kp_{12}$ = 111—113°C) in 86%iger Ausbeute in das reine Heptandiol-3,4 überführt wurde.

### Beispiel 6

In einer Apparatur gemäß Beispiel 1 wurde 1 Mol (=129 g) 2-Äthyl-Hexen-2-ol-I mit 0,7 Mol Ameisensäure (98%; 33 g) vermischt und bei 55°C im Verlauf von 4 Stunden mit 1,5 Mol $H_2O_2$ (20%ig; 255 g) versetzt. Der Ansatz wurde danach noch 4 Stunden bei 60°C weitergerührt und blieb auch dabei 2-phasig. Die wäßrige Phase wurde abgetrennt, und die organische Phase wurde zweimal mit Wasser (je 50 ml) nachgewaschen.

Alle vereinigten wäßrigen Phasen wurden katalytisch von $H_2O_2$ befreit und mit 65 g NaOH (40%ig) neutral gestellt. Die wäßrige Phase wurde danach erschöpfend mit Äthylacetat extrahiert. Der Äthylacetatextrakt wurde mit der organischen Phase vereinigt und destillativ von Leichtsiedern befreit. Das verbleibende Sumpfprodukt wog 143 g und ließ sich durch Hochvakuumdestillation (0,1 Torr; 90—92°C) in reines 2-Äthyl-hexantriol-1,2,3 überführen.

### Patentansprüche

1. Verfahren zur Hydroxylierung von 3-Chlor-2-methylpropen-1, von geradkettigen oder verzweigten, unsubstituierten oder bereits durch 1 bis 2 Hydroxylgruppen substituierten Monoolefinen mit 4 bis 7 Kohlenstoffatomen und endständiger oder innenständiger Doppelbindung, von geradkettigen oder verzweigten, unsubstituierten oder bereits mit 1 bis 2 Hydroxylgruppen substituierten Monoolefinen mit 8 bis 10 Kohlenstoffatomen und innenständiger Doppelbindung oder von geradkettigen oder verzweigten Diolefinen mit 4 bis 10 Kohlenstoffatomen, dadurch gekennzeichnet, daß man das zu hydroxylierende Olefin bei einer Temperatur zwischen 30 und 80°C mit weniger als 2 Mol Ameisensäure und weniger als 2 Mol Wasserstoffperoxid, jeweils pro Mol zu hydroxylierender Doppelbindung, umsetzt, Ameisensäure mit einer Konzentration zwischen 20 und 100 Gewichtsprozent und Wasserstoffperoxid mit einer Konzentration von weniger als 50 Gewichtsprozent einsetzt, und die Konzentration des Wasserstoffperoxids in der wäßrigen Phase des Reaktionsgemisches während der Gesamtdauer der Umsetzung unter 15 Gewichtsprozent hält.

2. Verfahren nach Anspruch 1, dadurch gekennzeichnet, daß man die Umsetzung bei einer Temperatur zwischen 45 und 60°C vornimmt.

3. Verfahren nach Anspruch 1 oder 2, dadurch gekennzeichnet, daß man die Ameisensäure in einer Menge von 0,2 bis 0,8 Mol pro Mol zu hydroxylierender Doppelbindung einsetzt.

4. Verfahren nach einem der Ansprüche 1 bis 3, dadurch gekennzeichnet, daß man das Wasserstoffperoxid in einer Menge von 1,1 bis 1,5 Mol pro Mol zu hydroxylierender Doppelbindung einsetzt.

5. Verfahren nach einem der Ansprüche 1 bis 4, dadurch gekennzeichnet, daß man Wasserstoffperoxid mit einer Konzentration von 15 bis 40 Gewichtsprozent einsetzt.

## Claims

1. A process for the hydroxylation of 3-chloro-2-methylpropene-1, of straight- or branched-chain monoolefins which are unsubstituted or are already substituted by 1 or 2 hydroxyl groups and have 4 to 7 carbon atoms and a terminal or internal double bond, of straight- or branched-chain monoolefins which are unsubstituted or are already substituted by 1 or 2 hydroxyl groups and have from 8 to 10 carbon atoms and an internal double bond, or of straight- or branched-chain diolefins having from 4 to 10 carbon atoms, characterised in that the olefin to be hydroxylated is reacted at a temperature of from 30 to 80° C with less than 2 mols of formic acid and less than 2 mols of hydrogen peroxide, in each case per mol of double bond to be hydroxylated, formic acid of a concentration of from 20 to 100% by weight is used and hydrogen peroxide of a concentration of less than 50% by weight is used, and the concentration of the hydrogen peroxide in the aqueous phase of the reaction mixture is maintained below 15% by weight for the complete duration of the reaction.

2. A process according to claim 1, characterised in that the reaction is carried out at a temperature of from 45 to 60° C.

3. A process according to claim 1 or 2, characterised in that the formic acid is used in a quantity of from 0.2 to 0.8 mols per mol of double bond to be hydroxylated.

4. A process according to one of claims 1 to 3, characterised in that the hydrogen peroxide is used in a quantity of from 1.1 to 1.5 mols per mol of double bond to be hydroxylated.

5. A process according to one of claims 1 to 4, characterised in that hydrogen peroxide of a concentration of from 15 to 40% by weight is used.

## Revendications

1. Procédé pour hydroxyler le 3-chloro-2-mé-thyl-propène-1, des mono-oléfines à chaîne droite ou ramifiée, non substituée ou déjà substituée par 1 à 2 groupes hydroxyle, avec 4 à 7 atomes de carbone et une double liaison en bout de chaîne ou en millieu de chaîne, des mono-oléfines à chaîne droite ou ramifiée, non substituées ou déjà substituées par 1 à 2 groupes hydroxyle, avec 8 à 10 atomes de carbone et une liaison double en milieu de chaîne, ou des dioléfines à chaîne droite ou ramifiée avec 4 à 10 atomes de carbone, procédé caractérisé en ce que l'on fait réagir l'oléfine à hydroxyler à une température comprise entre 30 et 80°C avec moins de 2 moles d'acide formique et moins de 2 moles d'eau oxygénée par mole de double liaison à hydroxyler, que l'on met en oeuvre de l'acide formique avec une concentration de 20 à 100% en poids et de l'eau oxygénée avec une concentration de moins de 50% en poids, et que l'on maintient la concentration de l'eau oxygénée dans la phase aqueuse, pendant toute la durée de la réaction, à une valeur inférieure à 15% en poids.

2. Procédé selon la revendication 1, caractérisé en ce que l'on effectue la réaction à une température comprise entre 45 et 60°C.

3. Procédé selon l'une des revendications 1 ou 2, caractérisé en ce que l'acide formique est utilisé en une proportion de 0,2 à 0,8 mole par mole de double liaison à hydroxyler.

4. Procédé selon l'une quelconque des revendications 1 à 3, caractérisé en ce que l'eau oxygénée est utilisée en une proportion de 1,1 à 1,5 mole par mole de double liaison à hydroxyler.

5. Procédé selon l'une quelconque des revendications 1 à 4, caractérisé en ce que l'eau oxygénée est utilisée à une concentration de 15 à 40% en poids.